(19) Europäisches Patentamt
European Patent Office
Office européen des brevets

(11) Veröffentlichungsnummer: **0 198 172**
**B1**

(12) # EUROPÄISCHE PATENTSCHRIFT

(45) Veröffentlichungstag der Patentschrift: **16.05.90**

(21) Anmeldenummer: **86102051.9**

(22) Anmeldetag: **18.02.86**

(51) Int. Cl.$^5$: **A 61 K 31/355** //
(A61K31/355, 31:12)

(54) **Pharmazeutische Zubereitung.**

(30) Priorität: **02.04.85 DE 3512054**

(43) Veröffentlichungstag der Anmeldung:
**22.10.86 Patentblatt 86/43**

(45) Bekanntmachung des Hinweises auf die Patenterteilung:
**16.05.90 Patentblatt 90/20**

(84) Benannte Vertragsstaaten:
**AT BE CH DE FR GB IT LI LU NL SE**

(56) Entgegenhaltungen:

**CHEMICAL ABSTRACTS, Band 102, Nr. 20, 20. Mai 1985, Seite 387, Nr. 172657k, Columbus, Ohio, US; & JP - A - 60 01 125**

(73) Patentinhaber: **HERMES Fabrik pharmazeutischer Präparate Franz Gradinger GmbH & Co.**
**Georg-Kalb-Strasse 5-8**
**D-8023 Grosshesselohe (DE)**

(72) Erfinder: **Reimann, Jürgen, Dr.**
**Hochkalterstrasse 2**
**D-8000 München 90 (DE)**
Der weitere Erfinder hat auf seine Nennung verzichtet

(74) Vertreter: **Reinhard, Skuhra, Weise**
**Friedrichstrasse 31**
**D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft eine neue pharmazeutische Zubereitung gemäß dem Oberbegriff des anliegenden Patentanspruchs 1.

Ubichinone sind der Oberbegriff für verschiedene, in der Natur vorkommende Antioxidantien mit teilweise unterschiedlichen Wirkungsspektren. Dabei ist das folgende Ubichinon

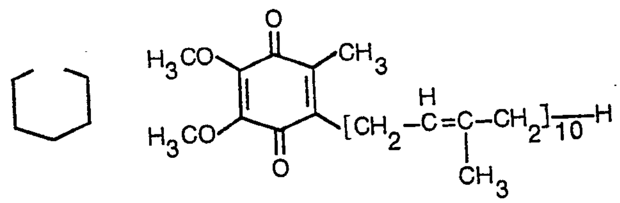

für den Menschen spezifisch. Dabei handelt es sich also um das 2.3-Dimethoxy-5-methyl-1.4-benzochinon mit einer isoprenoiden Seitenkette, mit 10 Dihydroisopren-Einheiten.

Das vorstehende Ubichinon, das im folgenden mit $CoQ_{10}$ abgekürzt wird, ist ein direktes Glied der mitochondrialen Atmungskette, die in jedem Muskel vorkommt. Die höchste Konzentration wurde in den Mitochrondien des Herzmuskels gefunden. Dies ist auch der Grund dafür, daß $CoO_{10}$ bereits als Therapeutikum in der Herztherapie eingesetzt wurde.

Aus "Drugs Expt. Clin.Res. X(7) Seiten 503—512 (1984)"ist es bereits bekannt, $CoQ_{10}$ zur Erhöhung des Herz-Zeit-Volumens einzusetzen.

Die Vitamin E-Gruppe ist eine Sammelbezeichnung für fettlösliche, natürlich vorkommende Verbindungen mit einem Chromangrunderüst und einer $C_{16}$-Seitenkette. Das wichtigste Vitaman E ist das allerdings nicht in der Natur vorkommende D,L-α-Tocopherol bzw. dessen Acetat.

Es ist schließlich bekannt, das Vitamin E als Therapeutikum einzusetzen. Die Wirkungen von Vitamin E sind ausführlich in dem Standardwerk von Machlin "Vitamin E—A comprehensive treatise" Verlag Marcel Dekker (1980 beschrieben.

Aus der japanischen Patentanmeldung JP—A—60 01 125 ist eine Zusammensetzung aus D,L-α-Tocopherol und Ubidecarenon bekannt. Das Tocopherol wird hier zur foto-oxidativen Stabilisierung des Ubidecarenons verwendet. Diese anti-oxidative Eigenschaft kommt ausschließlich den freien Tocopherolen zu und nicht den Tocopherol-estern. Deshalb sind in der JP—A—60 01 125 auch die Ester des Tocopherols explizit ausgenommen.

Obwohl bekannt ist, daß $CoQ_{10}$ herzaktive Eigenschaften besitzt, weist es doch den großen Nachteil auf, daß diese Wirkungen im Vergleich zu anderen bekannten Herzpräparaten (z.B. Digitalis) ausgesprochen schwach sind. Außerdem stellt es nach wie vor ein großes Problem dar, $CoQ_{10}$ großtechnisch in reinster Form wirtschaftlich herzustellen.

Eine Aufgabe der Erfindung ist es daher, diese Nachteile zu vermeiden, insbesondere eine pharmazeutische Zubereitung zu schaffen, die auf der Basis einer physiologischen und untoxischen Arzneimittelkombination ausgeprägte kardiotone Wirkungen besitzt.

Diese Aufgabe wird erfindungsgemäß dadurch gelöst, daß die pharmazeutische Zubereitung als Wirkstoffe $CoQ_{10}$ der Formel

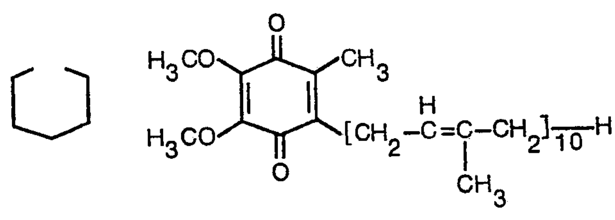

und D,L-α-Tocopherol-acetat im Gewichtsverhältnis 1:2 bis 1:100, sowie gegebenenfalls zusätzlich übliche Träger-, Verdünnungs- und/oder Zusatzstoffe enthält.

Vorzugsweise beträgt das Gewichtsverhältnis der beiden Wirkstoffe $CoQ_{10}$ und D,L-α-Tocopherol-acetat 1:3 bis 1:40.

Signifikante Ergebnisse wurden bei Gewichtsverhältnissen von 1:3 und 1:40 erzielt.

Die Herstellung der erfindungsgemäßen pharmazeutischen Zubereitung geschieht in an sich bekannter Weise dadurch, daß die beiden Wirkstoffe zunächst im angegebenen Verhältnis z.B. in einem Loedige- oder Diosna-Mischer gemischt und gegebenenfalls unter Zusatz der üblichen Träger-, Verdünnungs- und/oder Zusatzstoffe zu einer festen, oralen Darreichungsform geformt werden.

Insbesondere kann die pharmazeutische Zubereitung dadurch hergestellt werden, daß Weichgelatinekapseln gefertigt werden, beispielsweise folgendermaßen: Die beiden Wirkstoffe werden wie vorstehend beschrieben zunächst gemischt, mit öligen, physiologisch unbedenklichen Substanzen (z.B. Sojabohnenöl) gemischt und in Weichgelatinebänder eingebettet.

Bei $CoQ_{10}$ handelt es sich um einen physiologischen, körpereigenen Stoff bzw. bei D,L-α-Tocopherol-acetat um eine mit den entsprechenden körpereigenen Stoffen eng verwandte und physiologisch vergleichbare Substanz. Beide haben keine toxikologische Relevanz.

Die mittlere Dosierung beträgt unter Berücksichtigung der vorstehenden Gewichtsverhältnisse vorzugsweise 1—3 mal täglich eine orale Darreichungsform.

Als pharmazeutische Formen kommen insbesondere die orale Verabreichung, aber auch die intravenöse, intramuskuläre und parenterale Verabreichung in Betracht.

Für seine orale Verabreichung kann die erfindungsgemäße pharmazeutische Zubereitung in verschiedenen pharmazeutischen Formen angeboten werden, nämlich in festem oder gelöstem Zustand oder im Gemisch mit inerten Bestandteilen, mit oder ohne die üblichen Zusätze, wie färbende Mittel, Duftstoffe, Süßstoffe und andere analoge Produkte.

Im folgenden werden einige nichtbeschränkende Beispiele von Rezepturen für vorzugsweise Darreichungsformen offenbart:

Wenn nicht anders angegeben, sind dabei die Prozentangaben in Gewichtsprozent und die Grad-Angaben in Grad Celsius

1. *Tropfen*

| | |
|---|---|
| Vitamin-E-acetat | 10,0 % |
| $CoQ_{10}$ | 3,3 % |
| Polyethylenglykol 40 — hydriertes Rizinusöl (Cremophor® RH 40, BASF) | 24,0 % |
| 1,2-Propylenglykol | 4,0 % |
| Wasser | ad 100,0 % |

Vitamin E und $CoQ_{10}$ mit Polyethylenglykol 40 — hydriertes Rizinusöl und 1,2-Propylenglykol bei 60° mischen und das Wasser (35°) langsam einrühren.

2. *Tabletten*

| | |
|---|---|
| Vitamin-E-acetat Trockenpulver 50% coated (BASF) | 20,0 kg |
| $CoQ_{10}$ | 3,3 kg |
| Calciumhydrogenphosphat-Dihydrat (Parmcompress®) | 13,0 kg |
| hochdisperse Kieselsäure (Syloid® 74) | 4,0 kg |
| Polyvinylpyrrolidon (Kollidon® CL) | 2,5 kg |

Alle Substanzen durch Sieb 0,5 mm geben, eine 1/2 Stunde im Kubusmischer mischen und auf einem Rundläufer zu Tabletten pressen: Tablettendurchmesser 12 mm, Tablettengewicht 475 mg.

3. *Pulver*

| | |
|---|---|
| Vitamin-E-acetat Trockenpulver 50% coated (BASF) | 20,0 kg |
| $CoQ_{10}$ | 3,3 kg |
| hochdisperses Siliciumdioxid (Aerosil®) | 0,5 kg |
| Milchzucker | 10,0 kg |
| Saccharin-Na | 0,2 kg |
| Magnesium-stearat | 2,5 kg |

Die aufgeführten Substanzen werden miteinander gemischt und in Einzelpulver abgeteilt konfektioniert.

4. *Granulat*

| | |
|---|---|
| Vitamin-E-acetat Trockenpulver 50% coated (BASF) | 20,0 kg |
| $CoQ_{10}$ | 3,3 kg |
| Milchzucker | 15,0 kg |
| hochdisperses Siliciumdioxid (Aerosil®) | 0,5 kg |
| Methylcellulose | 5,0 kg |
| Saccharin-Natrium | 0,2 kg |
| Polyvinylpyrrolidon 5%-ige wäßrige Lösung, q.s. | 5,0 kg |

Die einzeln aufgeführten Substanzen werden miteinander gemischt, mit der Polyvinylpyrrolidon-Lösung befeuchtet und durch ein Sieb der Größe 1,5—2,5 mm gegeben und anschließend zum Trocknen ausgelegt.

5. *Kautabletten*

| | |
|---|---|
| Vitamin-E-acetat Trockenpulver 50% coated (BASF) | 100,0 kg |
| $CoQ_{10}$ | 33,0 kg |
| Mannit | 117,0 kg |
| hochdisperses Siliciumdioxid (Aerosil® 200) | 1,5 kg |
| Saccharin-Natrium | 0,1 kg |
| Magnesium-stearat | 3,0 kg |

Alle Bestandteile im Kubusmischer 15 Minuten lang mischen und zu Tabletten mit folgenden Eigenschaften verpressen:
Tablettendurchmesser 12 mm, Tablettengewicht ca. 700 mg.

6. *Brausetabletten*

| | | |
|---|---|---|
| I | Natriumhydrogencarbonat | 100,0 kg |
| | Weinsäure | 86.0 kg |
| II | Polyvidonacetat (Kollidon® VA64) | 2,0 kg |
| | Methylenchlorid | 10,0 kg |
| | Isopropanol | 10,0 kg |
| III | Vitamin-E-acetat Trockenpulver SD 50% (BASF) | 145,0 kg |
| | $CoQ_{10}$ | 48,0 kg |
| | Lactose | 82,0 kg |
| | Polyethylenglykol 6000 (Pulver) | 20,0 kg |
| | hochdisperses Siliciumdioxid (Aerosil® 200) | 5,0 kg |

Mischung I mit Lösung II granulieren, durch ein Sieb mit 0,8 mm Maschenweite geben, nach dem Trocknen Mischung III zugeben, mischen und bei einer Pulverfeuchte von ca. 30% zu Tabletten zu folgenden Eigenschaften verpressen:
Tablettendurchmesser 12 mm, Tablettengewicht ca. 625 mg.

7. *Kapseln* (pro Kapsel):

| | |
|---|---|
| Vitamin-E-acetat | 100,0 mg |
| $CoQ_{10}$ | 33,0 mg |
| Polysorbat 80 — Polyethylenglykolsorbitan-oleat | 3,0 mg |
| Triglyceride, gesättigt q.s. für eine Kapselfüllmenge von 280 mg | |

Die Stoffe werden miteinander gemischt und, wie bereits vorher beschrieben, in Weichgelatinekapseln abgefüllt.

Die erfindungsgemäße pharmazeutische Zubereitung ist insbesondere bei den folgenden Indikationen angezeigt:

1. Bessere Sauerstoffverwertung und damit eine Erhöhung des Herz-Zeit-Volumens (das Herz-Zeit-Volumen ist diejenige Menge Blut in Litern, die in einer Minute das Herz passiert; CO = Cardiac output) und damit verbunden eine Erhöhung des Schlagvolumens (SV).

2. Allgemeine Leistungssteigerung im körperlichen und geistigen Bereich.

3. Verbesserung der Mikrozirkulation im peripheren, im coronaren und im zerebralen Bereich.

4. Intakterhaltung von Zellmembranen, auf Basis spezifischer Wirkungen als Antioxidantien.

5. Schutz gegen exogene und endogene Radikale und Radikalbildner, z.B. $O^{2-}$, Singulett-Sauerstoff $^{1}O$, Peroxide und Ozon.

Im folgenden werden klinische Versuche am Menschen beschrieben, die die Wirkungen der erfindungsgemäßen pharmazeutischen Zubereitung zeigen:

Tabelle 1 zeigt in schematischer Darstellung eine Doppel-Blind-Studie mit der erfindungsgemäßen pharmazeutischen Zubereitung.

Dabei bedeutet "I.C."Impedanz Cardiographie.

Zur Durchführung der Impedanz Cardiographie wird insbesondere verwiesen auf "Biomedical and clinical aspects for coenzyme Q, Volume 1 (1977), Volume 2 (1980), Volume 3 (1981), Volume 4 (1984), Elsevier/North-Holland Biomedical Press, Amsterdam-New York-Oxford" und auf Lee E. Baker (ebenda, Vol. 2, Seite 289) und schließlich W. G. Kubicek, Aerospace Med. 37 (12) 1208, 1966.

Tabelle 1

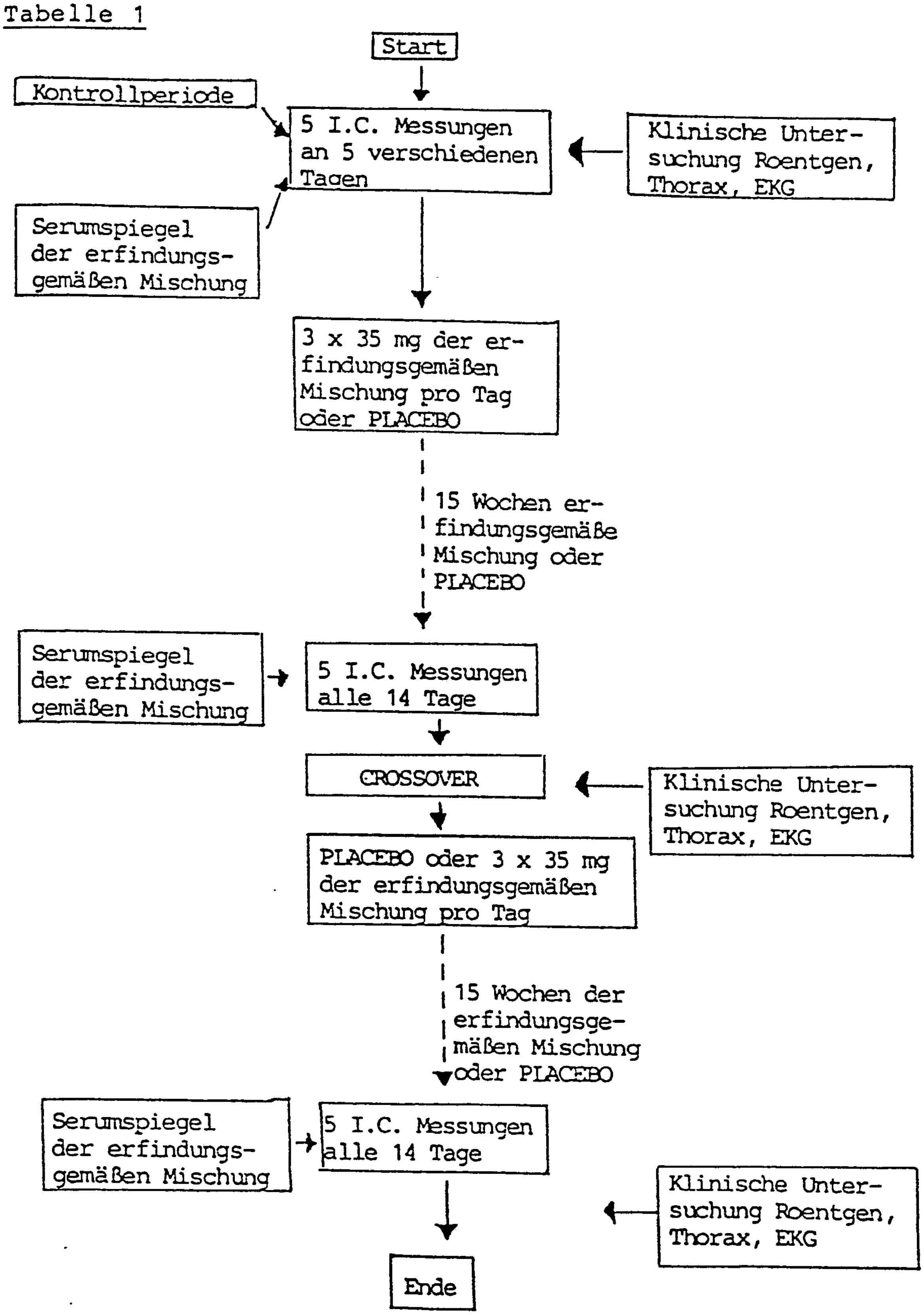
Start
Kontrollperiode
5 I.C. Messungen an 5 verschiedenen Tagen
Klinische Untersuchung Roentgen, Thorax, EKG
Serumspiegel der erfindungsgemäßen Mischung
3 x 35 mg der erfindungsgemäßen Mischung pro Tag oder PLACEBO
15 Wochen erfindungsgemäße Mischung oder PLACEBO
Serumspiegel der erfindungsgemäßen Mischung
5 I.C. Messungen alle 14 Tage
CROSSOVER
Klinische Untersuchung Roentgen, Thorax, EKG
PLACEBO oder 3 x 35 mg der erfindungsgemäßen Mischung pro Tag
15 Wochen der erfindungsgemäßen Mischung oder PLACEBO
Serumspiegel der erfindungsgemäßen Mischung
5 I.C. Messungen alle 14 Tage
Klinische Untersuchung Roentgen, Thorax, EKG
Ende

Die Wirkungen der erfindungsgemäßen pharmazeutischen Zubereitung auf das Herz-Schlag-Volumen (SV), das Herz-Zeit-Volumen (CO) und den Heather Index (HI) von sechs Patienten wurden ermittelt und in der folgenden Tabelle 2 zusammengestellt. Dabei wurden hochsignifikante Verbesserungen der genannten drei Einzelwerte gefunden.

Den sechs Patienten wurden gemäß Tabelle II gegeben:

| | |
|---|---|
| $CoQ_{10}$ | : 3 × 33 mg = 99 mg täglich |
| Placebo | : Oleum arachidis Vol. (eine Kapsel 0,5 ml) |
| $CoQ_{10}$ + Vitamin E-acetat | : (3 × 33 mg) + (3 × 100 mg) zusammen in 3 Kapseln täglich gelöst in Oleum arachidis |
| Vitamin E-acetat | : 3 × 100 mg täglich (= 3 Kapseln täglich). |

TABELLE 2

Stand. Mittelwerte des Herzminuten volumens (CO), des Schlagvolumens (SV) und des Heather Indexes (III) an 6 Patienten mit Myopathia cordis, Stadium II—III (nach NYHA) Herzinsuffizienzen. Nach Gaben von $CoQ_{10}$, Placebo, $CoQ_{10}$ + Vit-E-acetat und Vitamin E-acetat alleine über 30 Wochen. Doppel-Blind-Studie mit Crossover, siehe Tabelle 1. Ermittelt mit Impedanz Cardiographen, Surcom, USA (IC)

| 1 | 2 | 3 | 4 | 5 | 6 |
|---|---|---|---|---|---|
| Parameter | Kontrollperiode 5 IC-Messungen je 25 Werte pro Messung | Placebo 15 Wochen 7 IC-Messungen je 25 Werte pro Messung | $CoQ_{10}$ allein 15 Wochen 3 × 33 mg tgl. 7 IC Messungen je 25 Werte pro Messung | $CoQ_{10}$+Vit.E-acetat 3 × 33 mg $CoQ_{10}$+ 3 × 100 mg Vit.E-acetat 7 IC-Messungen je 25 Werte pro Messung | Vit.E-acetat allein 3 × 100 mg Vit.E-acetat* |
| CO | 3,2 L (st.d.±0,2) | 3,2 L (st.d.±0,2) | 3,9 L (st.d.±0,2) | 6,1 L (st.d.±0,3) | 3,3 L (st.d.±0,2) |
| SV | 44,0 ml (st.d.±0,3) | 45,0 ml (st.d.±0,2) | 69 ml (st.d.±0,2) | 131 ml (st.d.±0,3) | 45,5 ml (st.d.±0,2) |
| HI | 10,0 ml (st.d.±0,1) | 10,0 ml (st.d.±0,2) | 7,8 ml (st.d.±0,1) | 5,23 ml (st.d.±0,1) | 10,5 ml (st.d.±0,2) |

*Optovit, Hermes, München L=Liter; st.d. = "standard deviation" = Abweichung

Aus Tabelle 2 sieht man deutlich, daß $CoQ_{10}$ allein (Spalte 4) eine etwa 20 %ige Steigerung des Herzminutenvolumens (CO) gegenüber den Kontrollen (2), den Placebo's (3) und Vitamin E-acetat alleine (6) bewirkt.

Ähnliches gilt für das Schlagvolumen (SV) und den Heather Index (HI).

In Spalte 5 ($CoQ_{10}$ + Vit. E-acetat) wird der synergistische — bzw. potenzierende — Effekt von Vitamin E-acetat auf $CoQ_{10}$ eindeutig sichtbar.

Das Herzminutenvolumen wird auf nahezu 100% erhöht. Ähnliches gilt für das Schlagvolumen (SV) und den Heather Index (HI).

Obwohl aus der Literatur bereits seit längerem bekannt ist, daß Vitamin E und die Ubichinone ganz allgemein ähnliche biophysikalische Wirkungsmechanismen im Organismus besitzen im Sinne von Elektronenüberträgereigenschaften bei biologischen Oxidationsprozessen, war nicht zu erwarten, daß eine Kombination der Wirkstoffe $CoQ_{10}$ und D,L-α-Tocopherol -acetat von vornherein sinnvoll sei, und zwar aus folgenden Gründen: Bei $CoQ_{10}$ ist seit geraumer Zeit bekannt, daß es, in der Therapie angewandt, das Herzmuskelvolumen [siehe Biomedical and Clinical Aspects of Coenzyme Q (Band 1—3), Elsevier/North Holland Biomedical Press, Amsterdam, New York, Oxford (1977, 1981, 194)] erhöht. Diese Eigenschaften sind in der gesamten Literatur für das Vitamin E und das Vitamin-E-acetat jedoch nicht beschrieben. Aus diesem Grunde wäre höchstens eine Kombination mit anderen kardial-wirksamen Stoffen wie z.B. Crataegus, Digitalis oder Strophantus sinnvoll und theoretisch zu erwarten gewesen, aber auf keinen Fall eine Kombination mit Vitamin-E-acetat. Obwohl also ähnliche biophysikalische Wirkungen für beide Stoffe beschrieben werden, bestehen wesentliche Unterschiede in den pharmakologischen und pharmakodynamischen Eigenschaften der beiden Stoffe. Erst in der Kombination von Vitamin-E-acetat mit $CoQ_{10}$ zeigten sich erfindungsgemäß überadditive synergistische Effekte auf die Steigerung des Herzminutenvolumens. Gleichzeitig wurde ein $CoQ_{10}$-einsparender Effekt ("sparing effect") erzielt. Außerdem handelt es sich, trotz unterschiedlicher Wirkungsmechanismen, bei $CoQ_{10}$ um eine physiologische, körpereigene Substanz bzw. bei D,L-α-Tocopherol-acetat (Vitamin-E-acetat) um eine mit den entsprechenden körpereigenen Stoffen eng verwandte und physiologisch vergleichbare Substanz. Beide Wirkstoffe ergänzen sich nicht nur in Bezug auf ihre biochemischen Wirkungen (diese sind bekannt), sondern auch in Bezug auf ihre pharmakologischen und pharmakodynamischen Eigenschaften in ihrer Kombination synergistisch, ja sie potenzieren sich sogar gegenseitig. Die Wirkungen der erfindungsgemäßen pharmazeutischen Zubereitung, insbesondere ihr synergistischer Effekt, waren auch in Kenntnis der Einzelwirkungen in hohem Maße überraschend und nicht zu erwarten.

**Patentansprüche für die Vertragsstaaten: BE CH DE FR GB IT LI LU NL SE**

1. Pharmazeutische Zubereitung, enthaltend $CoQ_{10}$ der Formel

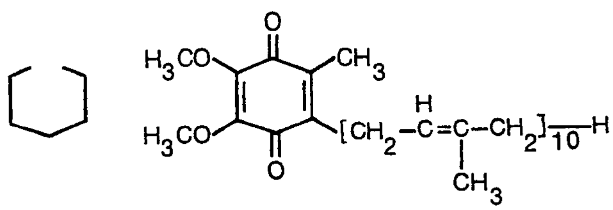

dadurch gekennzeichnet, daß sie als Wirkstoffe $CoQ_{10}$ und D,L-α-Tocopherol-acetat im Gewichtsverhältnis 1:2 bis 1:100, sowie gegebenenfalls zusätzlich übliche Träger-, Verdünnungs- und/oder Zusatzstoffe enthält.

2. Pharmazeutische Zubereitung nach Anspruch 1, dadurch gekennzeichnet, daß die beiden Wirkstoffe im Gewichtsverhältnis 1:3 bis 1:40 vorliegen.

3. Pharmazeutische Zubereitung nach Anspruch 1 oder 2, dadurch gekennzeichnet, daß die beiden Wirkstoffe im Gewichtsverhältnis 1:3 vorliegen.

4. Pharmazeutische Zubereitung nach Anspruch 1 oder 2, dadurch gekennzeichnet, daß die beiden Wirkstoffe im Gewichtsverhältnis 1:40 vorliegen.

5. Verwendung der pharmazeutischen Zubereitung nach einem der Ansprüche 1 bis 4 zur Herstellung eines Arzneimittels zur Erzielung einer besseren Sauerstoffverwertung und damit einer Erhöhung des Hertz-Zeit-Volumens (CO) und einer Erhöhung des Schlagvolumens (SV).

6. Verwendung der pharmazeutischen Zubereitung nach einem der Ansprüche 1 bis 4 zur Herstellung eines Arzneimittels zur Erzielung einer allgemeinen Leistungssteigerung im körperlichen und geistigen Bereich.

7. Verwendung der pharmazeutischen Zubereitung nach einem der Ansprüche 1 bis 4 zur Herstellung eines Arzneimittels zur Erzielung einer Verbesserung der Mikrozirkulation im peripheren, im coronaren und im zerebralen Bereich.

8. Verwendung der pharmazeutischen Zubereitung nach einem der Ansprüche 1 bis 4 zur Herstellung eines Arzneimittels zur Intakterhaltung von Zellmembranen.

9. Verwendung der pharmazeutischen Zubereitung nach einem der Ansprüche 1 bis 4 zur Herstellung eines Arzneimittels zum Schutz gegen exogene und endogene Radikale und Radikalbildner.

**Patentansprüche für den Vertragsstaat: AT**

1. Verfahren zur Herstellung einer pharmazeutischen Zubereitung auf der Basis einer physiologischen und untoxischen Arzneimittelkombination mit ausgeprägter kardiotoner Wirkung, dadurch gekennzeichnet, daß die Wirkstoffe $CoQ_{10}$ der Formel

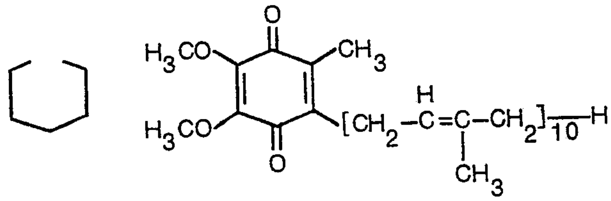

und D,L-α-Tocopherol-acetat im Gewichtsverhältnis 1:2 bis 1:100 in einem Mischer gemischt und gegebenenfalls unter Zusatz der üblichen Träger, Verdünnungs- und/oder Zusatzstoffe zur pharmazeutischen Zubereitung geformt werden.

2. Verfahren nach Anspruch 1, dadurch gekennzeichnet, daß die beiden Wirkstoffe im Gewichtsverhältnis 1:3 bis 1:40 gemischt werden.

3. Verfahren nach Anspruch 1 oder 2, dadurch gekennzeichnet, daß die beiden Wirkstoffe im Gewichtsverhältnis 1:3 gemischt werden.

4. Verfahren nach Anspruch 1 oder 2, dadurch gekennzeichnet, daß die beiden Wirkstoffe im Gewichtsverhältnis 1:40 gemischt werden.

5. Verfahren nach einem der vorhergehenden Ansprüche, dadurch gekennzeichnet, daß 10 Gew.-% Vitamin-E-acetat mit 3,3 Gew.% $CoQ_{10}$ und 24 Gew.% Polyethylenglykol 40-hydriertes Rizinusöl und 4,0 Gew.% 1,2-Propylenglykol bei 60°C gemischt werden und Wasser bei einer Temperatur von 35°C in einer Menge ad 100 Gew.% langsam in diese Mischung eingerührt wird.

**Revendications pour les Etats contractants: BE CH DE FR GB IT LI LU NL SE**

1. Préparation pharmaceutique contenant du $CoQ_{10}$ de formule

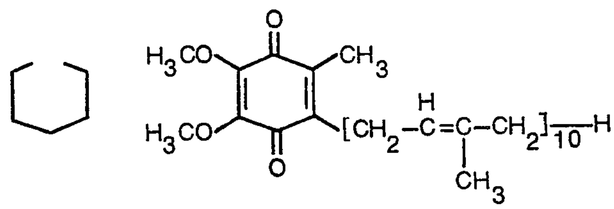

caractérisée en ce que, comme matières actives, elle contient du $CoQ_{10}$ et de l'acétate de D,L-α-tocophérol dans un rapport en poids allant de 1:2 à 1:100, ainsi qu'en plus, le cas échéant, des matières de support, de dilution et/ou d'addition usuelles.

2. Préparation pharmaceutique selon la revendication 1, caractérisée en ce que les deux matières actives sont présentes dans un rapport en poids allant de 1:3 à 1:40.

3. Préparation pharmaceutique selon la revendication 1 ou 2, caractérisé en ce que les deux matières actives sont présentes dans un rapport en poids de 1:3.

4. Préparation pharmaceutique selon la revendication 1 ou 2, caractérisée en ce que les deux matières actives sont présentes dans un rapport en poids de 1:40.

5. Utilisation de la préparation pharmaceutique selon l'une des revendications 1 à 4, pour la réalisation d'un médicament destiné à assurer une meilleure utilisation de l'oxygène et donc une augmentation du débit cardiaque périodique (DC) et une augmentation du volume d'éjection (VE).

6. Utilisation de la préparation pharmaceutique selon l'une des revendications 1 à 4, pour la réalisation d'un médicament destiné à assurer une augmentation de rendement générale dans les domaines corporel et intellectuel.

7. Utilisation de la préparation pharmaceutique selon l'une des revendications 1 à 4, pour la réalisation d'un médicament destiné à assurer une amélioration de la microcirculation dans les régions périphérique, coronarienne et cérébrale.

8. Utilisation de la préparation pharmaceutique selon l'une des revendications 1 à 4, pour la réalisation d'un médicament destiné à conserver l'intégrité des membranes cellulaires.

9. Utilisation de la préparation pharmaceutique selon l'une des revendications 1 à 4, pour la réalisation d'un médicament destiné à assurer une protection contre des radicaux et des formateurs de radicaux exogènes et endogènes.

**Revendications pour l'Etat contractant: AT**

1. Procédé de fabrication d'une préparation pharmaceutique, sur la base d'une combinaison physiologique et non toxique de produits pharmaceutiques, possédant un effet cardiotonique prononcé, caractérisé en ce que les matières actives $CoQ_{10}$ de formule

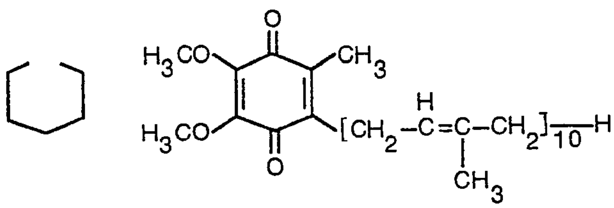

et acétate de D,L-α-tocophérol sont mélangées dans un mélangeur dans un rapport en poids allant de 1:2 à 1:100 et, le cas échéant, sont mises en forme par l'addition, à la préparation pharmaceutique, de matières de support, de dilution et/ou d'addition usuelles.

2. Procédé selon la revendication 1, caractérisé en ce que les deux matières actives sont mélangées dans un rapport en poids allant de 1:3 à 1:40.

3. Procédé selon la revendication 1 ou 2, caractérisé en ce que les deux matières actives sont mélangées dans un rapport en poids de 1:3.

4. Procédé selon la revendication 1 ou 2, caractérisé en ce que les deux matières actives sont mélangées dans un rapport en poids de 1:40.

5. Procédé selon l'une des revendications précédentes, caractérisé en ce que, à 60°C, on mélange 10% en poids d'acétate de vitamine E, 3,3% en poids de $CoQ_{10}$, 24% en poids de polyéthylèneglycol 40-huile de ricin hydrogénée et 4,0% en poids de 1,2 propylèneglycol et l'on dilue lentement ce mélange avec de l'eau à une température de 35°C et en quantité suffisante pour atteindre 100% en poids.

**Claims for the Contracting States: BE CH DE FR GB IT LI LU NL SE**

1. Pharmaceutical preparation containing $CoQ_{10}$ of the formula

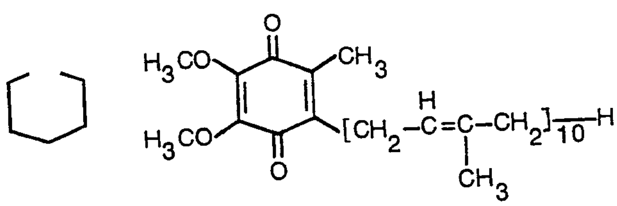

characterized in that it contains as active substances $CoQ_{10}$ and D,L-α-tocopherol acetate in the weight ratio 1:2 to 1:100 and possibly additionally usual carrier, dilution and/or additive substances.

2. Pharmaceutical preparation according to claim 1, characterized in that the two active substances are present in the weight ratio 1:3 to 1:40.

3. Pharmaceutical preparation according to claim 1 or 2, characterized in that the two active substances are present in the weight ratio 1:3.

4. Pharmaceutical preparation according to claim 1 or 2, characterized in that the two active substances are present in the weight ratio 1:40.

5. Use of the pharmaceutical preparation according to any one of claims 1 to 4 for producing a drug for obtaining a better oxygen utilization and thus an increase in the cardiac volume (Co) and an increase in the stroke volume (SV).

6. Use of the pharmaceutical preparation according to any one of claims 1 to 4 for producing a drug for obtaining a general increase in vitality in the physical and mental area.

7. Use of the pharmaceutical preparation according to any one of claims 1 to 4 for producing a drug for obtaining an improvement in the microcirculation in the peripheral, coronary, and cerebral area.

8. Use of the pharmaceutical preparation according to any one of claims 1 to 4 for producing a drug for maintaining cell membranes intact.

9. Use of the pharmaceutical preparation according to any one of claims 1 to 4 for producing a drug for protection against exogenous and endogenous radicals and radical formers.

**Claims for the Contracting State: AT**

1. Method for producing a pharmaceutical preparation on the basis of a physiological and nontoxic

drug combination with pronounced cardiotonic effect, characterized in that the active substances $CoQ_{10}$ of the formula

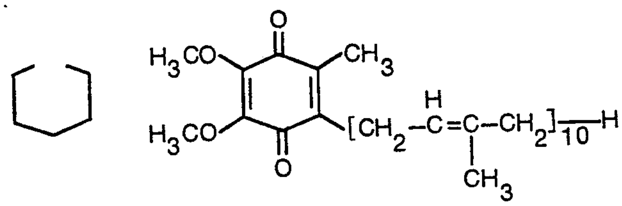

and D,L-α-tocopherol acetate are mixed in the weight ratio 1:2 to 1:100 in a mixer and possibly under addition of the usual carrier, dilution and/or additive substances formed to the pharmaceutical preparation.

2. Method according to claim 1, characterized in that the two active substances are mixed in the weight ratio 1:3 to 1:40.

3. Method according to claim 1 or 2, characterized in that the two active substances are mixed in the weight ratio 1:3.

4. Method according to claim 1 or 2, characterized in that the two active substances are mixed in the weight ratio 1:40.

5. Method according to any one of the preceding claims, characterized in that 10% by weight vitamin E acetate with 3.3% by weight $CoQ_{10}$ and 24% by weight polyethylene glycol 40-hydrogenated caster oil and 4.0% by weight 1,2 propylene glycol are mixed at 60°C and water stirred into said mixture slowly at a temperature of 35°C in an amount to make up 100% by weight.